# EUROPEAN PATENT SPECIFICATION

(11) **EP 0 662 841 B1**
(45) Date of publication and mention of the grant of the patent: **16.09.1998**
(21) Application number: 93923685.7
(22) Date of filing: 05.10.1993
(51) Int. Cl.: A61K 47/48, A61K 38/49

(54) **PHARMACEUTICAL COMPOSITION FOR SITE-SPECIFIC RELEASE OF A CLOT-DISSOLVING PROTEIN**
PHARMAZEUTISCHE ZUSAMMENSETZUNG FÜR ORTSSPEZIFISCHE ABGABE VON BLUTGERINSELAUFLÖSENDE PROTEINE
COMPOSITION PHARMACEUTIQUE PREVUE POUR LA LIBERATION SPECIFIQUE SUR LE SITE D'UNE PROTEINE DISSOLVANT LES CAILLOTS DE SANG

(30) Priority: 05.10.1992 NL 9201722
(43) Date of publication of application: 19.07.1995
(73) Proprietor: Stichting voor de Technische Wetenschappen, 3502 GA Utrecht (NL)
(72) Inventor: HEEREMANS, Johanna, Louise, Maria, NL-3564 BD Utrecht (NL); KLUFT, Cornelis, NL-2171 VE Sassenheim (NL); GROMMELIN, Daniel, Jan, Anne, NL-3583 VP Utrecht (NL)
(74) Representative: de Bruijn, Leendert C.
(86) International application number: NL9300195
(87) International publication number: WO9407537

(56) References cited:
- EP-A- 0 227 400
- EP-A- 0 310 065
- WO-A-90/07924
- WO-A-91/05545
- BE-A- 831 867
- GB-A- 2 021 262
- STN FILE SERVER & FILE MEDLINE AN=90194057 (KARLSRUHE) & THROMB. RES. vol. 57, no. 3 , 1 February 1990 pages 333 - 42 HR. LIJNEN ET AL. 'EFFECT OF FIBRIN-TARGETING ON CLOT LYSIS WITH UROKINASE-TYPE PLASMINOGEN ACTIVATOR.'
- CHEMICAL ABSTRACTS, vol. 118, no. 16, 19 April 1993, Columbus, Ohio, US; abstract no. 154308y, & PROC. INT. SYMP. CONTROLLED RELEASE BIOACT. MATER. 19TH 1992 pages 62 - 62 J.L. HEEREMANS ET AL 'DEVELOPMENT OF A PROCEDURE FOR COUPLING THE HOMING DEVICE GLU-PLASMINOGEN TO LIPOSOMES.'
- BIOCHIM. BIOPHYS. ACTA vol. 1117, no. 3 , 1992 pages 258 - 264 J. L. HEEREMANS ET AL. 'DEVELOPMENT OF A PROCEDURE FOR COUPLING THE HOMING DEVICE GLU-PLASMINOGEN TO LIPOSOMES.'
- Fibrinolysis 1994,8 (suppl. 2), 138-141

## Description

The invention relates to a pharmaceutical composition for the release of a clot-dissolving protein at the site of a blood clot.

Acute myocardial infarcts are usually caused by a blood clot completely or substantially blocking one of the coronary arteries. Blood clots frequently also play a role in other acute or chronic abnormalities in the blood circulation. It is known to use clot-dissolving proteins in the treatment of such conditions. Said proteins lead directly or indirectly to thrombolysis, via conversion of plasminogen present in the blood to plasmin, which in turn may decompose fibrin. However, the administration of clot-dissolving proteins has the disadvantage that thrombolysis also occurs elsewhere and plasminogen activation takes place in the blood circulation, as a result of which haemorrhaging and thrombo-embolic complications can arise. In order to avoid systemic effects of this type it is therefore necessary that the clot-dissolving proteins are not able to exert their action in the entire blood circulation but only at the site of the threatening clot. Various proposals have already been made in order to achieve this specificity by encapsulating the clot-dissolving protein and providing the whole with a blood clot-seeking substance.

WO-A-90/07924 discloses a method of coupling targeting molecules such as immunoglobulins, containing a free amino group, to liposomes which are provided with a thiol function, which coupling is performed by a bifunctional agent, succinimidyl 3-(2-pyridyldithio)propionate, reacting with the amino and thiol groups. The liposomes may contain a diagnostic agent.

A liposome composition consisting of liposomes modified with a hydrophilic polymer such as polyethyleneglycol or polylactic acid, in which a medicament such as an antidiuretic, anticoagulant, tPA or anti-tumour agent can be present, is disclosed in WO-A-91/05545.

In WO-A-90/14078 an injectable preparation is described for the treatment of thrombosis, which preparation comprises semipermeable or decomposable microcapsules (liposomes) containing a plasminogen activator in an aqueous medium in the core thereof. It is stated in this publication that the liposomes are preferably "site-specific", but no measures are proposed for such a target-directed release.

BP-A-450,479 and EP-A-363,712 disclose a bispecific monoclonal antibody which, on the one hand, is able to bind a thrombolytic agent, such as tissue-type plasminogen activator, and, on the other hand, is specific for activated blood platelets or fibrin; this antibody is used to control blood clots.

These known medicaments have the disadvantage that they are not sufficiently specific and/or are not supplied sufficiently effectively at the site of the blood clots, or that the agents are not inherent in the body and thus are potentially immunogenic.

The object of the invention is to provide a pharmaceutical composition and a method for the preparation thereof, with which blood clots can be effectively and safely treated.

Surprisingly, it has been found that the natural interaction between plasminogen and plasminogen activator can, even at high molar ratios of activator over plasminogen, be reduced in such a way that a particulate composition can be prepared which contains a plasminogen activator as clot-dissolving protein and plasminogen as targeting device, the intended functions of both plasminogen activator and plasminogen remaining intact.

The invention therefore relates to a pharmaceutical composition which contains a biodegradable colloidal carrier with a targeting device which shows affinity for the blood clot and with a plasminogen activator as clot-dissolving protein, the targeting device being plasminogen bonded to the colloidal carrier.

The biodegradable colloidal carrier is in particular a liposome. As is usual, liposomes are understood to be microvesicles built up of phospholipids and/or sterols and optionally glycolipids and other constituents, which vesicles enclose a water-containing compartment. The phospholipids can also partly or wholly be modified with hydrophilic polymers, such as polyethyleneglycol. The microvesicles can be unilamellar or multilamellar or have other forms. The diameter of the carrier particles is not critical and is in general in the range from 20 nm to 10 µm, more particularly between 100 nm and 1 µm. Other biodegradable colloidal carrier systems such as polycyanoacrylate, gelatin or albumin nanoparticles may also be used.

Variation in the nature of the biodegradable colloidal carrier, in particular of the liposome bilayer structure (e.g. "gel" or "fluid" state bilayers containing saturated or unsaturated fatty acid residues respectively, or presence of cholesterol in the bilayer), allows tuning of the release characteristics and of the clearance in the human or animal body, of the pharmaceutical composition of the invention according to the specific use. For example, acute cardiac infarcts require a quick clot lysis, and thus a quick release of the thrombolytic agent. Short circulation times are then sufficient. On the other hand, the treatment deep-venous thrombosis calls for relatively long-term, low-level administration of thrombolytic. Modification of the colloidal carrier e.g. with polyethyleneglycol will result in a composition which can be injected in a single dose over 24 hours.

Suitable plasminogen activators are urokinase, prourokinase, streptokinase, staphylokinase, streptokinase-plasminogen complex, tissue-type plasminogen activator (tPA, fibrinokinase), animal plasminogen activators, such as vampire bat plasminogen activator, and similar activators, as well as chimeric, mutated, truncated and chemically modified analogues of such plasminogen activators. Preferably, the composition according to the invention comprises a naturally occurring plasminogen activator and/or a plasminogen activator of human origin, more particularly a naturally occurring thrombolytic agent, such as tPA. An advantage of tPA is that, under physiological conditions, it has a high specificity and a high affinity for all types of fibrin.

Naturally occurring plasminogen, in particular glu-plasminogen, such as glu1-plasminogen and glu2-plasminogen, is preferably used as targeting device for the composition according to the invention. Plasminogen derivatives, such as lys-plasminogen and mini-plasminogen, can optionally also be used. As far as possible, the plasminogen is in a conformation suitable for reaction with fibrin.

It has been found that the coupling of plasminogen to the carrier particles can be improved if plasminogen is used which is substantially free from impurities such as primary amines. In particular, the plasminogen to be used contains less than 140 mol, especially less than 70 mol of free primary amines, such as ε-amino-caproic acid (EACA), per mole of plasminogen. More preferably, the plasminogen contains less than 20 mol, preferentially less than 13.3 mol of primary amines per mol of plasminogen; all assuming standard performance of the coupling reaction at around 6.5 mg plasminogen per ml, and requiring proportional lower contamination when higher plasminogen concentrations are used. In case of plasminogen preparations which initially contain more than e.g. 13.3 mol EACA per mol of plasminogen, the required low concentrations of primary amines can be obtained by special purification steps, for example gel filtration using a column which has a large overcapacity. When such purification steps are used, plasminogen is also found to be obtained in a favourable conformation for recognition of fibrin.

The carrier loading can vary, depending on the intended use. Advantageously, liposomes contain at least 10⁻⁶ and more preferentially at least 10⁻⁵ µmol of plasminogen activator per µmol of phospholipid (one µmol of phospholipid corresponding to approximately 750 µg). The plasminogen activator is essentially located on the inside of the liposomes and/or in the lipid bilayer, but can in part also be present on the outer surface.

The plasminogen which acts as targeting device is preferably chemically bonded to the outer surface of the carrier. The carrier particles each contain more than one plasminogen molecule, so that the affinity constant of the plasminogen-carrier adduct with respect to fibrin is greater than that of free plasminogen. Preferably, each carrier particle has, on average, at least 10 plasminogen molecules, More particularly, 10-1000 plasminogen molecules are bound per carrier particle. Expressed in µmol of plasminogen per µmol of phospholipid, the loading is preferably 1.6×10⁻⁵ to 1.6×10⁻³.

The biodegradable colloidal carrier of the composition according to the invention can also contain other agents, additives or auxiliaries, such as other thrombolytic agents, or agents which control the speed of release in vivo or the stability of the carrier in vitro. For example, the liposomes can be hypertonic, so that the release of the thrombolytic agent at the intended site is accelerated by osmotic pressure differences.

The compositions according to the invention can be in diverse forms, such as aqueous dispersions or powders. Freeze-dried powders are particularly suitable for long term storage. A cryoprotectant, such as an amino acid or a sugar, can then also be present. The composition can also contain acceptable excipients, such as an aqueous solution, salts, stabilisers and other auxiliaries. The compositions can also contain non-encapsulated plasminogen activator.

The pharmaceutical compositions can be used for the treatment of various pathological processes, in particular in man. These uses include the treatment of (acute) myocardial infarction, deep-venous thrombosis, pulmonary embolisms and arterial occlusions and also fibrin formation without occluding thrombus.

The composition can be administered in a manner known per se, such as intravenously, intra-arterially, intramuscularly, intra-peritoneally, dermally, subcutaneously and the like. The amounts and concentrations to be used can be determined by a person skilled in the art, partly on the basis of the biological availability at the site of the thrombus, and on the basis of physical parameters such as body weight, state of health and age.

When administering the compositions described above, it is also possible, at the same time or beforehand, to administer non-encapsulated plasminogen activator in order to achieve a combined effect, that is to say a rapid preparatory effect of the non-encapsulated activator ("bolus"), and a slower effect of the encapsulated activator.

The release of tPA at the site of the clot can be influenced, not only by the presence of release-controlling substances, but also by destabilisation of the complex by blood components, by a change in the distribution of the plasminogen over the carrier material or by complement-mediated breakdown.

The invention also relates to a method for the preparation of a composition which comprises a biodegradable colloidal carrier with a targeting device which shows affinity for the blood clot, in which method:
a) plasminogen which is substantially free from primary amines is bound to the colloidal carrier, the affinity of the plasminogen for fibrin being essentially maintained; and
b) if desired, a medicament or a diagnostic agent is incorporated in the colloidal carrier.

In particular, the plasminogen comprises less than 70 mol of free amines per mol of plasminogen as described above.

The compositions prepared in this way can be used as such. However, a thrombolytic agent or another medicament, or a diagnostic agent such as a radioisotope, a gas bubble or another detectable material, can also be incorporated in the carrier in the method.

The invention also relates to a method for the preparation of a pharmaceutical composition for the treatment of blood clots as described above.

The preferred variant of the method according to the invention comprises the following steps:
a) plasminogen is bound to liposomes, the affinity of the plasminogen for fibrin being essentially maintained; and
b) a plasminogen activator is incorporated in the liposomes under conditions such that the interaction of the plasminogen with the plasminogen activator is reduced.

The liposomes to be used in the method according to the invention can be prepared in a manner known per se, for example via the "film" method as described by F. Szoka and D. Papahadjopoulos, *Ann*. *Rev*. *Biophys*. *Bioeng*. 9, 467-508 (1980), the "ethanol injection" method (S. Batzri and E.D. Korn, *Biochim*, *Biophys*. *Acta* 298, 1015 (1973)), or the "ether injection" method (D.W. Deamer and A.D. Bangham, *Biochim*, *Biophys*. *Acta* 394, 483 (1976)), or by preparation of "reversed phase evaporation vesicles" (F. Szoka and D. Papahadjopoulos, *Proc*. *Natl*. *Acad*. *Sci*. *USA*, 75, 4194 (1978)). The liposomes prepared in this way can be unilamellar or multilamellar. Other liposomes or biodegradable colloidal carriers and other methods for the preparation thereof can also be used.

In step a) the plasminogen is bound, preferably covalently, to the colloidal carrier. The bonding of plasminogen to the carrier can, for example, take place on the basis of the reaction of a thiol group introduced in the protein with an activated double bond present in the carrier, or in another manner known per se for the coupling of peptides.

In the case of binding to liposomes by means of a thiol reaction, which is found to lead to good results, the following sub-steps of step a) can be differentiated:
a1) conversion of the plasminogen into a mercapto group-containing derivative, for example a mercaptoacyl derivative: in this step free amino groups in the protein, such as lysine residues, are converted to derivatives; a suitable agent for this purpose is succinimidyl acetylthioacetate (SATA), which after coupling and deacetylation (using hydroxylamine) leads to a mercaptoacetyl derivative of the protein (see R.J.S. Duncan et al., *Anal*. *Biochem*. 132, 68-73 (1983), and R.A. Schwendener et al., *Biochim*. *Biophys*. *Acta* 1026, 69-79 (1990)); when a plasminogen is used which is largely free from amines such as ε-aminocaproic acid, it is possible to introduce 3 to 9 thiol groups per molecule of plasminogen, whereas only 0 to 1.8 thiol groups can be introduced when commercially available glu-plasminogen is used;
a2) preparation of liposomes which are provided with anchor groups which are able to react with a mercapto group; suitable anchor groups of this type are, for example, maleimido groups; to this end, a suitable maleimido-phosphatidyl compound can be incorporated in the phospholipid components; a suitable example of such a compound is [4-(p-maleimidophenyl)butyryl]phosphatidylethanolamine (MPB-PE) (see F.J. Martin et al., *J*. *Biol*. *Chem*. **257**, 286-288 (1982));
a3) reaction of the plasminogen derivative with the liposomes provided with anchor groups; in the case of the example mentioned above, the reaction is then the reaction of the thiolated plasminogen with the liposomes provided with maleimido groups; this reaction can proceed by incubation in a suitable buffer; and
a4) separation of plasminogen bound to liposomes from non-bound plasminogen, for example by ultracentrifuge.

Another suitable method of coupling plasminogen to liposomes consists in modifying the liposomes with a hydrophilic polymer (as described in WO-A-91/05545), followed by coupling of plasminogen to the ends of the polymer chains. The hydrophilic polymer can be polyethylene glycol, e.g. having a molecular weight of between 2,000 and 5,000, which can be bound to the liposomes by covalent bonds using e.g. a polyethyleneglycol having terminal carboxyl groups and distearylphosphatidylethanolamine and a coupling activator such as a carbodiimide. Plasminogen can then be coupled to the modified liposomes using a coupling activator such as a carbodiimide.

Other methods for binding plasminogen to the colloidal carrier can also be used in the method according to the invention. A usable method is, for example, coupling by means of glutaraldehyde to, on the one hand, hydroxyl or amino groups of the protein (plasminogen) and, on the other hand, hydroxyl or amino groups of the carrier, as described by V.P. Torchilin et al., *Biochem*. *Biophys*. *Res*. *Commun*, **85**, 983-990 (1978). The avidin-biotin reaction, as described by D. L. Urdal and S. Hakomori, *J*. *Biol*. *Chem*. **255**, 10509-10516 (1980), can also be used, in which reaction avidin is conjugated to the carrier and can be bound to a biotinylated protein. In addition, use can be made of the method described by A.A. Bogdanov et al., *FEBS Letters* **231**, 381-384 (1988) according to which a protein on the carrier surface can be immobilised by activation with a carbodiimide in the presence of N-hydroxysulfosuccinimide. Variants of these techniques and other techniques, such as via disulphide bridge formation, can also be used. The coupling of the plasminogen must, as far as possible, be carried out in such a way that the ability of the plasminogen to bind with fibrin remains intact.

In the case of coupling by means of thioether formation, the colloidal carrier is preferably treated with a thiol, such as cysteine, prior to step b), in order to protect the residual anchor groups on the carrier against binding of the plasminogen activator. This step can be carried out before or after a4).

In step b) the plasminogen and/or the plasminogen activator are treated in such a way that the conversion of plasminogen by plasminogen activator is reduced or blocked, in other words in such a way that plasminogen is not converted, or is converted to only a small extent, into plasmin by free plasminogen activator, including in cases where relatively little substrate (plasminogen) and a relatively large amount of enzyme (plasminogen activator) are present. This treatment is preferably also reversible, so that the plasminogen can be converted by the plasminogen activator at the site of a blood clot. It has been found that the conversion of plasminogen by plasminogen activator can be reduced by using a low temperature and/or a low pH, in particular a pH of about 4 (3 to 5). Other agents can also be used for this purpose, such as a metal halide, for example zinc chloride, or an aminoalkanoic acid having 5-7 carbon atoms, for example ε-aminocaproic acid (EACA) or ornithine. The affinity of plasminogen for fibrin, which is necessary for recognition of the clot and which may be reduced during the coupling, is regained after administration of the composition.

In step b) the plasminogen activator can be incorporated in the colloidal carrier in a manner known for the encapsulation of proteins (for incorporation in liposomes, see for example, C. Kirby and G. Gregoriadis, *Biotechnology* 2, 979 (1984) or M.B. Bally et a. *Biochim*, *Biophys*. *Acta* **812**, 66 (1985)). It is a physical encapsulation which is involved here.

It has been found that tPA can be incorporated in relatively large amounts by freeze-thawing of the liposomes in the presence of the plasminogen activator. Preferably, the freeze-thawing is carried out several times. Prior to freeze-thawing, the liposomes are taken up in a suitable buffer, to which a surface-active substance, such as Tween® 80, has optionally been added, in order to prevent losses. The buffer has a pH of about 3 to 8, preferably about 4. The concentrations and amounts used for the encapsulation are also determined by the desired composition of the end product.

Although the sequence of procedures described above is preferred, the clot-decomposing composition according to the invention can also be prepared using a method in which:
c) a plasminogen activator is incorporated in a biodegradable colloidal carrier and
d) plasminogen is bound to the colloidal carrier, the affinity of the plasminogen for fibrin being essentially maintained, if necessary under conditions such that the interaction of the plasminogen with the plasminogen activator is reduced.
In this method, phospholpids or other carrier components can first be provided with anchor groups, after which the latter can be reversibly protected, for example by means of mercapto compounds. The carrier particles are then formed and the plasminogen activator encapsulated, after which the protection is removed from the anchor groups. If the plasminogen activator is completely encapsulated in the carrier, the treatment described above for lowering the interaction with its activator can be completely or partly omitted. The plasminogen is then bound with the aid of a coupling agent to anchor sites in the carrier.

The compositions obtained in the manners described above can be further treated and made suitable for use. Preferably, the composition is freeze-dried after step a), before or after step b) or after step d). The freeze-drying can be carried out in the presence of a cryoprotectant, such as an amino acid e.g. arginine, or a sugar or sugar alcohol, e.g. lactose or mannitol. In this way a stable product is obtained which can be reconstituted before use and up to that time is not subject to the risks of leakage, decomposition or plasmin formation.

The invention also relates to combinations of compositions, termed "kits" below. One embodiment of such a kit comprises at least a pharmaceutical composition as described above and optionally further means for the preparation and administration of the composition. In addition, the kit can comprise a separate plasminogen activator, for combined administration.

In another embodiment the kit comprises at least a pharmaceutical composition which contains a biodegradable colloidal carrier which has a plasminogen bound thereto as targeting device, and thus is without encapsulated plasminogen activator, and a composition which contains a plasminogen activator. All or part of the plasminogen activator can be incorporated in the composition before use.

In yet another embodiment, the kit comprises, on the one hand, a composition containing a biodegradable colloidal carrier to which plasminogen low in free amine is coupled, and, on the other hand, a medicament or a diagnostic agent, which optionally can be incorporated in the colloidal carrier before use.

### Example I

### Purification of glu-plasminogen

Three ml portions of commercially available high-concentration glu-plasminogen solution containing 80 mg of protein were purified over a 1 meter column having a capacity of 100 ml and packed with Sephadex G25 medium using 10 mM HEPES buffer. The purification led to removal of 45 mol of ε-aminocaproic acid per mol of glu-plasminogen. This purification step was shown to increase the reactivity of the protein during the thiol-introducing SATA reaction (see below): whereas for the unpurified sample around 1 mol SH per mol of glu-plasminogen was introduced, this number was increased to 9 mol SH per mol of glu-plasminogen for the purified sample.

Addition of pure EACA to the purified glu-plasminogen sample accordingly caused a decrease in reactivity towards the SATA reaction, with a decrease to about half at 14 mol EACA per mol glu-plg and progressive decrease to 1 mol SH/mol and 0.1 mol SH/mol glu-plg at 10 and 100-fold higher EACA concentrations, respectively.

### Thiolation of plasminogen

73 µM of glu-plasminogen, dissolved in 10 mM HEPES buffer which contained 135 mM of sodium chloride and 1 mM of EDTA, were incubated for 20 minutes at room temperature at pH 7.5 with a solution of 584 µM of succinimidyl (acetylthio)acetate (SATA) in dimethylformamide (DMF), using a volume ratio of DMF : buffer of 1:100. The mixture was then separated on a Sephadex® G-50 column. The protein fractions were detected by measuring the absorption at 280 nm. The fractions were combined and stored at -20°C. Protein concentrations were determined using the methods of Wessel and Lowry (see: R.K. Scopes, Protein Purification; Principles and Practice, 2nd Ed., Springer (1987) 278-283).

The acetylthioacetyl protein (glu-plasminogen-ATA) was deacetylated by treatment with a freshly prepared solution of 0.5 M hydroxylamine.HCl in 0.5 M HEPES containing 25 mM EDTA (pH = 7.5); volume ratio of protein solution : hydroxylamine solution = 10:1. The concentration of free sulphydryl groups was then determined with the aid of 5,5'-dithiobis(2-nitrobenzoic acid) using the method of G.L. Ellman, *Arch*. *Biochem*. *Biophys*. **82**, 70-77 (1959). For this determination a blank was used which contained both protein not converted into a derivative and hydroxylamine, in order to avoid errors as a consequence of the inherent absorption of the hydroxylamine solution. For a SATA : protein ratio of 8:1, it was found to be possible to incorporate 0.5-9 SH groups per mol of glu-plasminogen, depending on whether commercial glu-plasminogen or purified glu-plasminogen was used.

### Preparation of liposomes

Liposomes provided with anchors for protein molecules were prepared using phosphatidylcholine, phosphatidylglycerol, cholesterol and [4-(p-maleidophenyl)butyryl]phosphatidylethanolamine (MPB-PE) in a molar ratio of 38.5:4:16:0.5-1.5 as starting materials, in accordance with the method of F. Szoka and D. Papahadjopoulos, *Ann*. *Rev*. *Biophys*. *Bioeng*. 9, 467-508 (1980). The MPB-PE was prepared using the method of F.J. Martin and D. Papahadjopoulos, *J*. *Biol*. *Chem*. **257** (1), 286-288 (1982). The liposomes were extruded through polycarbonate membranes having pores of 0.6 µm (1x), 0.4 µm (1x) and 0.2 µm (3x) in size. After extrusion, the average diameter (determined by dynamic light scattering) was 0.24 ± 0.01 µm. The liposomes were prepared in 10 mM HEPES containing 135 mM NaCl and 1mM EDTA. Phospholipid concentrations were determined, after destruction with perchloric acid, using the method of C.H. Fiske and Y. Subbarow, *J*. *Biol*. *Chem*. **66**, 375-400 (1925).

### Coupling of thiolated plasminogen to liposomes

Thioacetyl-glu-plasminogen was added in various amounts to the liposomes obtained and the mixture was incubated for 30, 75 or 120 minutes. The phospholipid concentration was 6.7 µmol/ml and the protein concentration 0.5 to 2.5 mg/ml in HEPES, pH 7.4. The incubation volume was 0.5 ml. The coupling reaction was terminated by adding 50 µl of N-ethylmaleimide (8 mM in HEPES) and the liposomes were separated from free protein by twice ultracentrifuging for 45 minutes at 80,000 x g at 4°C (Beckman Instruments). The activity of free glu-plasminogen, glu-plasminogen in derivative form and liposomal glu-plasminogen was determined by activation with streptokinase using the method of P. Friberger et al., *Haemostasis* **7**, 138-145 (1978). The plasminogen samples were incubated with a molar excess of streptokinase and the activity of the complex was determined on the basis of the conversion of the synthetic substrate S-2251 (H-D-Val-Leu-Lys-pNA), which was measured by means of the absorption of the reaction product pNA (paranitroanilide) at 405 nm. The activity of glu-plasminogen converted into the SATA derivative was found to be somewhat reduced compared with untreated glu-plasminogen. The enzymatic activity of glu-plasminogen bound to liposomes was found to be reduced to 68% (± 6%) of the original activity (that is to say before converting to the SATA derivative).

### Determination of binding of glu-plasminogen to fibrin

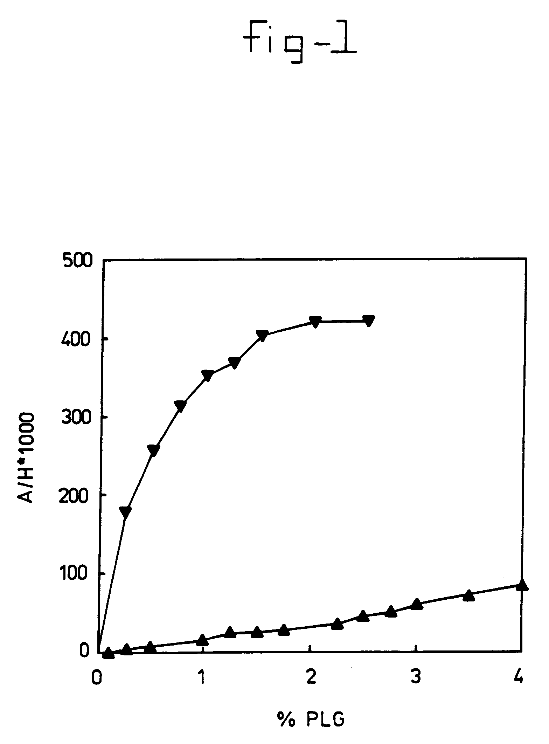

Titertek plates were covered with a layer of fibrinogen and then activated with thrombin, plates having a layer of fibrin monomers with a density of about 0.033 nmol/cm² being obtained. The plates obtained were treated with Tween® 20 in order to prevent non-specific binding of glu-plasminogen with untreated parts of the plate. Samples of 15 nmol/l glu-plasminogen (1% of the plasminogen concentration in plasma) were incubated overnight at room temperature at pH 7.4. The supernatant was collected and the bound fraction was washed three times with buffer. The plasminogen concentration in both fractions was determined with the aid of the conversion of S-2251 as described above. It was then possible to calculate the percentage of bound protein. The concentrations used for the incubation of liposomal glu-plasminogen were the same as those for the free protein. The buffer contained 0.05 M Tris-HCl, 0.85% NaCl and 0.01% by weight of Tween® 20. No appreciable degradation of the liposomes occurred with this Tween® concentration. The fibrin-binding capacity of glu-plasminogen coupled with liposomes was compared with that of free glu-plasminogen. The ratio of fibrin-bound protein to total protein was calculated for both forms of plasminogen for a fixed low concentration of 15 nmol/l of glu-plasminogen. The protein density of the liposome fraction was 50 µg per mol of phospholipid. The fraction of the liposome protein which was bound to fibrin was more than ten times as large as that of the free protein. This effect is shown in the appended figure 1. In this figure the binding of the plasminogen-liposome adduct ( 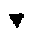 ) and that of the free plasminogen (▲) is plotted in absorption units per hour as a function of the plasminogen concentration. When empty liposomes were added to the free protein, no interaction with the binding to fibrin occurred.

### Example II

### Preparation of liposomes containing tissue-type plasminogen activator

The liposomes (prepared in accordance with the method of Example I) were suspended in concentrations of 75, 100 and 150 µmol/ml phospholipid in buffer (10 mM Tris, 135 mM NaCl, pH 7.5; or 0.1 N citrate, 0.264 M sucrose, pH 4; or 0.1 N citrate, 0.029 M sucrose, pH 4) in the presence or absence of 0.05% Tweed® 80. Tissue-type plasminogen activator (tPA) was added to the suspension in a concentration of 37, 92 and 185 µg/ml. The liposomes were then freeze-thawed five times in order to increase the encapsulacing capacity of the liposomes. Non-encapsulated tPA was removed by ultracentrifuging for 45 minutes at 150,000 x g at 4°C. During ultracentrifuging, 0.05% Tween® 80 was incorporated in the buffer in order to prevent loss of non-encapsulated tPA from the supernatant.

### Example III

### Preparation of liposomes with plasminogen as targeting device and containing tissue-type plasminogen activator

Plasminogen-containing and tPA-containing liposomes were prepared according to the procedures described in Examples I and II. In the absence of fibrin, the conversion rate of plasminogen by tPA is low. Further reduction of the conversion rate is achieved by using low pH (3-5) and/or by adding EACA (up to 7 mM) or metal halides.

### Example IV

### In vivo behaviour of liposomes containing tissue-type plasminogen activator

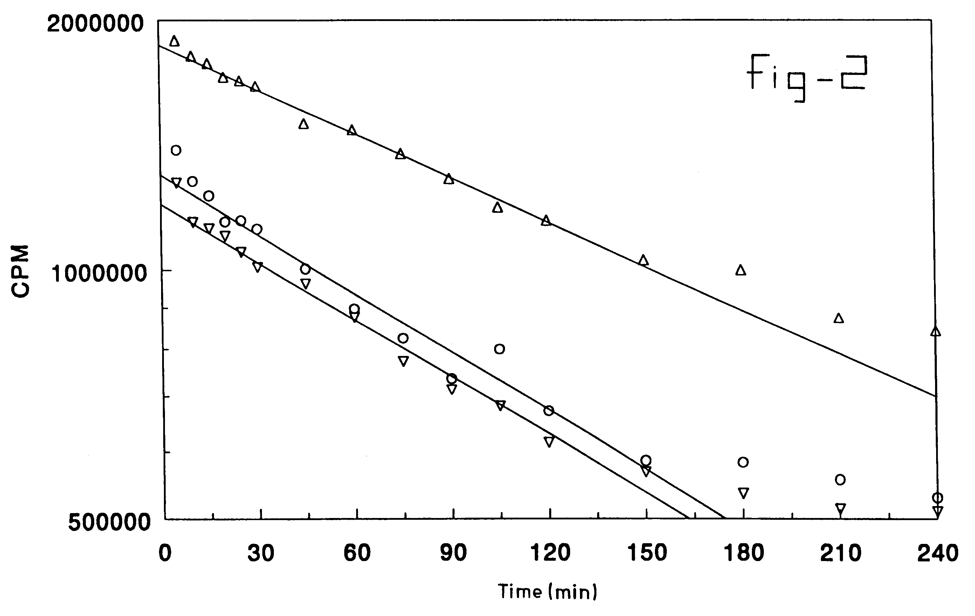

Liposomes containing tPA, as prepared according to Example II, the tPA being labelled with ¹²⁵I, were injected into three healthy rabbits (not having an artificial blood clot). A blood sample was taken every five minutes, and the tPA activity was determined after disruption of the liposomes with Tween® 80. The results are shown in Fig. 2. The three lines (△, ○, ∇) relate to the three rabbits. The average half-life of liposomal tPA appears to be about 140 minutes, which is substantially longer than for free tPA (about 2.5 minutes in rabbits), indicating containment of tPA in the liposomal compartment which clears slower than free tPA.

### Example V

### Liposomes with plasminogen bound to PEG-headgroups: plasminogen-containing liposomes with long circulation times in rats

Liposomes with plasminogen attached to PEG-DSPE (polyethylene-glycol-distearylphosphatidylethanolamine) groups were prepared by coupling DSPE to polyoxyethylene-bis(acetic acid) with 77 polyoxyethylene units (cf. Kung and Redemann, *Biochim*. *Biophys*. *Acta* **862**, 435-439 (1986)), and combining the coupling products with soybean phosphatidylcholine and cholesterol in a molar ratio of 5.4:67.6:27 (see also: G. Blame et al., *Biochim. Biophys*. Acta **1149**. 180-184 (1993). After intravenous injection into rats, relatively low clearance rates and long circulation times were found for these plasminogen-PEG-liposomes: twenty four hours after injection 18.4% of the initial dose of plasminogen-PEG-liposomes was still circulating compared to 4% for non-PEG-containing liposomes.

## Claims

1. Pharmaceutical composition for the treatment of blood clots, which composition comprises a biodegradable colloidal carrier containing a targeting device which shows affinity for a blood clot and containing a clot-dissolving protein, characterised in that the clot-dissolving protein is a plasminogen activator and the targeting device is plasminogen bound to the colloidal carrier.

2. Composition according to Claim 1, in which the plasminogen activator is tissue-type plasminogen activator (tPA).

3. Composition according to Claim 1 or 2, in which the plasminogen is glu-plasminogen.

4. Composition according to one of Claims 1-3, in which the biodegradable colloidal carrier is a liposome.

5. Composition according to Claim 4, which contains at least 10⁻⁶ µmol of plasminogen activator per µmol of liposome phospholipid.

6. Composition according to Claim 4 or 5, in which 10 to 1000 molecules of plasminogen are bound per liposome.

7. Method for the preparation of a composition which comprises a biodegradable colloidal carrier with a targeting device which shows affinity for the blood clot, in which method:
a) plasminogen from which free primary amines have been removed to the extent that it, at a plasminogen concentration of 6.5 mg/ml, contains less than 20 mol of free primary amines per mol of plasminogen, is bound to a biodegradable colloidal carrier, the affinity of the plasminogen for fibrin being essentially maintained; and
b) if desired, a medicament or a diagnostic agent is incorporated in the biodegradable colloidal carrier.

8. Method for the preparation of a composition having blood clot-decomposing properties, in which method:
a) plasminogen is bound to a biodegradable colloidal carrier, the affinity of the plasminogen for fibrin being essentially maintained; and
b) a plasminogen activator is incorporated in the biodegradable colloidal carrier under conditions such that the interaction of the plasminogen with the plasminogen activator is reduced.

9. Method according to Claim 7 or 8, in which, in step a), the plasminogen is bound by:
a1) conversion of the plasminogen into a mercapto group-containing derivative,
a2) preparation of a biodegradable colloidal carrier which is provided with anchor groups which are able to react with a mercapto group;
a3) reaction of the plasminogen derivative with the colloidal carrier provided with anchor groups; and
a4) separation of plasminogen bound to the biodegradable colloidal carrier from non-bound plasminogen.

10. Method according to Claim 9, in which the colloidal carrier is treated with a thiol, such as cysteine, prior to step b).

11. Method according to one of Claims 8-10, in which a pH of 3 to 8 is used in step b).

12. Method according to one of Claims 8-11, in which a metal halide and/or an aminoalkanoic acid is used in step b).

13. Method for the preparation of a composition having blood clot-decomposing properties, in which method:
c) a plasminogen activator is incorporated in a biodegradable colloidal carrier and
d) plasminogen is bound to the colloidal carrier, the affinity of the plasminogen for fibrin being essentially maintained, optionally under conditions such that the interaction of the plasminogen with the plasminogen activator is reduced.

14. Method according to one of Claims 8-13, in which, in step b) or step c) respectively, the plasminogen activator is incorporated in the colloidal carrier by freeze-thawing the carrier in the presence of the plasminogen activator.

15. Method according to one of Claims 7-14, in which, following steps a), b) or d), the composition is freeze-dried, optionally in the presence of a cryoprotectant.

16. Method according to one of Claims 7-15, in which the bio-degradable colloidal carrier is a liposome.

17. Kit at least comprising a pharmaceutical composition according to one of Claims 1-6 and a composition which contains a plasminogen activator.

18. Kit suitable for the treatment of blood clots, at least comprising a pharmaceutical composition which contains a biodegradable colloidal carrier, in particular a liposome, with a plasminogen bound thereto as targeting device and a composition which contains a plasminogen activator.

19. Kit at least comprising a composition as prepared using the method according to Claim 7, and a composition which contains a medicament or a diagnostic agent.

## Patentansprüche

1. Pharmazeutische Zusammensetzung zur Behandlung von Blutgerinnseln, wobei die Zusammensetzung einen eine Targeteinrichtung enthaltenden bioabbaubaren kolloidalen Carrier umfaßt, welcher eine Affinität für Blutgerinnsel zeigt und der ein Gerinnsel lösendes Protein enthalt,
**dadurch gekennzeichnet, daß**
das Gerinnsel auflösende Protein ein Plasminogen-Aktivator und die Targeteinrichtung ein an den kolloidalen Carrier gebundenes Plasminogen ist.

2. Zusammensetzung nach Anspruch 1, in welcher der Plasminogen-Aktivator ein Plasminogenaktivator vom Gewebetyp (tPA) ist.

3. Zusammensetzung nach Anspruch 1 oder 2, in welcher das Plasminogen Glu-Plasminogen ist.

4. Zusammensetzung nach einem der Ansprüche 1 bis 3, in welcher der bioabbaubare kolloidale Carrier ein Liposom ist.

5. Zusammensetzung nach Anspruch 4, welche zumindest 10⁻⁶ µmol des Plasminogen-Aktivators pro µmol des Liposomphospholipids enthält.

6. Zusammensetzung nach Anspruch 4 oder 5, in welcher 10 bis 1000 Plasminogenmoleküle pro Liposom gebunden sind.

7. Verfahren zur Herstellung einer Zusammensetzung, die einen bioabbaubaren kolloidalen Carrier mit einer Targeteinrichtung umfaßt, welche eine Affinität für das Blutgerinnsel zeigt, wobei in dem Verfahren:
a) Plasminogen, von dem freie primäre Amine in dem Ausmaß entfernt worden sind, so daß, bei einer Plasminogenkonzentration von 6,5 mg/ml, es weniger als 20 mol freie primäre Amine pro mol Plasminogen enthält, an einem biobabbaubaren kolloidalen Carrier gebunden wird, wobei die Affinität des Plasminogens für Fibrin im wesentlichen beibehalten, und
b) falls gewünscht, ein Arzneimittel oder ein Diagnosemittel in den bioabbaubaren kolloidalen Carrier eingearbeitet wird.

8. Verfahren zur Herstellung einer Zusammensetzung mit Blutgerinnsel zersetzenden Eigenschaften, wobei in dem Verfahren:
a) Plasminogen an einen bioabbaubaren kolloidalen Carrier gebunden, wobei die Affinität des Plasminogens für Fibrin im wesentlichen beibehalten wird, und
b) ein Plasminogen-Aktivator in den bioabbaubaren kolloidalen Carrier unter solchen Bedingungen eingearbeitet werden, daß die Interaktion des Plasminogens mit dem Plasminogen-Aktivator verringert wird.

9. Verfahren nach Anspruch 7 oder 8, in welchem in Schritt a) das Plasminogen durch
a1) Umwandlung des Plasminogens in ein mercaptogruppenhaltiges Derivat,
a2) Herstellung eines bioabbaubaren kolloidalen Carriers, welcher mit Ankergruppen versehen ist, welche in der Lage sind, mit einer Mercapto-Gruppe zu reagieren,
a3) Umsetzung des Plasminogenderivates mit dem mit Ankergruppen versehenen kolloidalen Carrier, und
a4) Abtrennung des an den bioabbaubaren kolloidalen Carrier gebundenen Plasminogens von nicht gebundenem Plasminogen
gebunden wird.

10. Verfahren nach Anspruch 9, in welchem der kolloidale Carrier mit einem Thiol, wie Cystein, vor dem Schritt b) behandelt wird.

11. Verfahren nach einem der Ansprüche 8 bis 10, in welchem ein pH von 3 bis 8 in Schritt b) verwendet wird.

12. Verfahren nach einem der Ansprüche 8 bis 11, in welchem ein Metallhalogenid und/oder eine Aminoalkansäure in Schritt b) verwendet wird.

13. Verfahren zur Herstellung einer Zusammensetzung mit Blutgerinnsel zersetzenden Eigenschaften, wobei in dem Verfahren:
c) ein Plasminogen-Aktivator in einen bioabbaubaren kolloidalen Carrier eingearbeitet, und
d) Plasminogen an den kolloidalen Carrier gebunden werden, wobei die Affinität des Plasminogens für Fibrin im wesentlichen aufrechterhalten wird, gegebenenfalls unter solchen Bedingungen, daß die Interaktion des Plasminogens mit dem Plasminogen-Aktivator verringert wird.

14. Verfahren nach einem der Ansprüche 8 bis 13, in welchem jeweils in dem Schritt b) oder Schritt c) der Plasminogen-Aktivator in den kolloidalen Carrier durch Gefrieren/Auftauen des Carriers in der Gegenwart des Plasminogen-Aktivators eingearbeitet wird.

15. Verfahren nach einem der Ansprüche 7 bis 14, in welchem im Anschluß an die Schritte a), b) oder d) die Zusammensetzung, gegebenenfalls in der Gegenwart eines Gefrierschutzmittels gefriergetrocknet wird.

16. Verfahren nach einem der Ansprüche 7 bis 15, in welchem der bioabbaubare kolloidale Carrier ein Liposom ist.

17. Ein zumindest eine pharmazeutische Zusammensetzung nach einem der Ansprüche 1 bis 6 und eine einen Plasminogen-Aktivator enthaltende Zusammensetzung aufweisender Kit.

18. Für die Behandlung von Blutgerinnsel geeigneter Kit, der zumindest eine pharmazeutische Zusammensetzung, die einen bioabbaubaren kolloidalen Carrier, insbesondere ein Liposom, mit einem daran gebundenen Plasminogen als Targeteinrichtung und eine Zusammensetzung, die einen Plasminogenaktivator enthält, umfaßt.

19. Zumindest eine unter Verwendung des Verfahrens nach Anspruch 7 hergestellte Zusammensetzung und eine Zusammensetzung, die ein Arzneimittel oder eine Diagnosemittel enthält, enthaltender Kit.

## Revendications

1. Composition pharmaceutique pour le traitement des caillots sanguins, ladite composition comprenant un support colloïdal biodégradable contenant un système de ciblage qui présente une affinité pour le caillot sanguin et contenant une protéine dissolvant le caillot caractérisée en ce que la protéine dissolvant le caillot est un activateur de plasminogène et le système de ciblage est du plasminogène fixé sur le support colloïdal.

2. Composition selon la revendication 1 dans laquelle l'activateur de plasminogène est un activateur de plasminogène de type tissulaire (EPA).

3. Composition selon la revendication 1 ou 2, dans laquelle le plasminogène est un glu-plasminogène.

4. Composition selon l'une quelconque des revendications 1 à 3, dans laquelle le support colloïdal biodégradable est un liposome.

5. Composition selon la revendication 4, qui contient au moins 10⁻⁶ µmole d'activateur de plasminogène par µmole de phospholipide de liposome.

6. Composition selon la revendication 4 ou 5, dans laquelle 10 à 1000 molécules de plasminogène sont liées par liposome.

7. Méthode de préparation d'une composition qui comprend un support colloïdal biodégradable avec un système de ciblage qui présente une affinité pour le caillot sanguin, dans laquelle :
a) du plasminogène dont les amines primaires libres ont été éliminées dans une mesure telle que, à une concentration de plasminogène de 6,5 mg/ml, il contient moins de 20 moles d'amines primaires libres par mole de plasminogène, est lié à un support colloïdal biodégradable, l'affinité du plasminogène pour la fibrine étant pratiquement conservée et
b) le cas échéant, un médicament ou un agent de diagnostic est incorporé dans le support colloïdal biodégradable.

8. Méthode de préparation d'une composition présentant des propriétés de décomposition des caillots sanguins, dans laquelle :
a) du plasminogène est fixé à un support colloïdal biodégradable, l'affinité du plasminogène pour la fibrine étant pratiquement conservée ; et
b) un activateur de plasminogène est incorporé dans le support colloïdal biodégradable dans des conditions telles que l'interaction du plasminogène avec l'activateur de plasminogène soit réduite.

9. Méthode selon la revendication 7 ou 8, dans laquelle, dans l'étape a), le plasminogène est fixé par :
a1) conversion du plasminogène en un dérivé contenant un groupe mercapto;
a2) préparation d'un support colloïdal biodégradable qui est muni de groupes d'ancrage qui sont capables de réagir avec un groupe mercapto ;
a3) réaction du dérivé de plasminogène avec le support colloïdal portant des groupes d'ancrage et
a4) séparation du plasminogène lié au support colloïdal biodégradable du plasminogène non lié.

10. Méthode selon la revendication 9, dans laquelle le support colloïdal est traité par un thiol, tel que la cystéine, avant l'étape b).

11. Méthode selon l'une quelconque des revendications 8 à 10, dans laquelle on utilise un pH de 3 à 8 dans l'étape b).

12. Méthode selon l'une quelconque des revendications 8 à 11, dans laquelle on utilise un halogénure de métal et/ou un acide amino-alcanoïque dans l'étape b).

13. Méthode de préparation d'une composition ayant des propriétés de décomposition des caillots sanguins, dans laquelle :
c) on incorpore un activateur de plasminogène dans un support colloïdal biodégradable ; et
d) on fixe le plasminogène sur le support colloïdal, l'affinité dit plasminogène pour la fibrine étant pratiquement conservée, éventuellement dans des conditions telles que l'interaction du plasminogène avec l'activateur de plasminogène soit réduite.

14. Méthode selon l'une quelconque des revendications 8 à 13, dans laquelle. dans l'étape b) ou l'étape c), respectivement, on incorpore l'activateur de plasmillogène dans le support colloïdal par congélation/décongélation du support en présence de l'activateur de plasminogène.

15. Méthode selon l'une quelconque des revendications 7 à 14, dans laquelle, après les étapes a), b) ou d), la composition est lyophilisée, éventuellement en présence d'un cryoprotecteur.

16. Méthode selon l'une quelconque des revendications 7 à 15, dans laquelle le support colloïdal biodégradable est un liposome.

17. Kit comprenant au moins une composition pharmaceutique selon l'une des revendications 1 à 6, et une composition qui contient un activateur de plasminogène.

18. Kit convenant au traitement des caillots sanguins, comprenant au moins une composition pharmaceutique qui contient un support colloïdal biodégradable, en particulier un liposome, avec un plasminogène fixé sur celui-ci en tant que système de ciblage et une composition qui contient un activateur de plasminogène.

19. Kit comprenant au moins une composition telle que préparée en utilisant la méthode selon la revendication 7, et une composition qui contient un médicament ou un agent de diagnostic.
